# EUROPEAN PATENT APPLICATION

(11) **EP 1 815 876 A2**
(43) Date of publication of application: **08.08.2007**
(21) Application number: 07002186.0
(22) Date of filing: 01.02.2007
(51) Int. Cl.: A61L 15/58, A61L 24/04, C09J 183/04

(54) **Hot-melt silicone based ostomy and wound care skin attachment adhesives**

(30) Priority: 02.02.2006 US 764395 P
(71) Applicant: Bristol-Myers Squibb Company, New York, N.Y. 10154 (US)
(72) Inventor: Sambasivam, Mahesh, Pennington NJ 08534 (US); Fattman, George F., Mt. Laurel NJ 08054 (US)
(74) Representative: Holmes, Miles Keeton

(57) **Abstract**

An ostomy or wound care appliance including an ostomy or wound care device for attachment to the body with an adhesive. The adhesive including a hotmelt silicone pressure sensitive adhesive composition and a reinforcing member.

## Description

### BACKGROUND OF THE INVENTION

The present invention is an ostomy or wound care appliance including an ostomy or wound care device for attachment to the body by a hot-melt pressure sensitive silicone adhesive.

There are several medical conditions that require attachment of a device to the body such as ostomy and wound care. These devices are typically attached to the body via a pressure sensitive adhesive. An ostomy appliance typically includes a wafer containing a pressure sensitive adhesive and a receiving bag to collect and contain feces. When the collecting bag is removably attached to wafer via a coupling mechanism, it is referred to as a two-piece device. When the wafer and the bag are permanently attached to each other, the device is referred to as a one-piece device. To a significant extent, one of the major performance characteristics of such devices is the adhesive bond to the skin.

These adhesives have to meet certain critical requirements such as: (i) bond to skin readily (tacky); (ii) easy to reposition; (iii) able to withstand load (high shear force); (iv) easy to remove (low peel force) and; (v) able to resist adhesion loss due to moisture (perspiration or during shower). The pressure sensitive adhesives used for attachment of ostomy devices to the body have been primarily hydrocolloid adhesives comprising a rubbery polymer matrix based on polyisobutylenes, styrenic block copolymers, or acrylics, blended with hydrocolloids. The rubbery polymer matrix provides the initial tack to adhere to the skin whereas the hydrocolloid provides moisture absorption and wet adhesion up to a certain extent. Some of the potential disadvantages of these hydrocolloid adhesives may be their: (i) stiffness; (ii) low tack; and (iii) poor performance under long term exposure to moisture. It should be noted that a typical ostomy wafer is worn for several days.

U.S. Patent No. 4,309,520 describes the composition of a silicone adhesive formulation which includes a silicone polymer, a silicone resin, and a silicone cluster compound. The addition of the cluster compound increases the tack characteristics of the adhesive.

WO 86/00532 describes a transdermal patch adhesive permeable to medicinal fluids through the epidermis comprising the polymerization product of aryl polysiloxane, an alkyl polysiloxane, and a MQ polysiloxane resin.

U.S. Patent No. 4,831,070 describes a moldable elastomeric pressure sensitive adhesive for attachment to skin comprising a xylene-soluble silicone resin copolymer, a polydiorganosiloxane fluid with hydroxyl and alkoxy end group, and a condensation catalyst.

U.S. Patent No. 4,865,920 describes the composition of hot-melt silicone adhesives comprising a silicate resin, a silicone fluid, and an organic ester.

EP 0261167 B1 describes a wound dressing comprising a soft hydrophobic silicone gel reinforced with a textile material or foam with perforations.

U.S. Patent No. 5,232,702 describes a silicone pressure sensitive adhesive composition compatible with drugs, exipients, and permeation enhancers comprising a silicone fluid, a silicate resin, and a cohesive strengthening agent such as a calcium stearate to prevent cold flow of the adhesive.

U. S. Patent No. 5,540,922 describes an absorbent wound dressing component comprising a silicone gel which is perforated, an absorbent body, and a backing substrate. The silicone gel adhesion to dry skin is described to be considerably lower than conventional tape adhesives used as wound dressings.

EP 0663430 B1 describes a hot-melt silicone adhesive suitable for delivery of hydrophilic drugs comprising a silicone resin, a silicone fluid, and a siloxylated allyloxypropane diol copolymer.

U.S. Patent Publication No. 2004/0102744 A1 describes silicone adhesives comprising a polysiloxane and a silicate resin suitable for attachment of ostomy pouches to an ostomy wafer to resist stomal effluent.

WO 2004/108175 A1 describes an elastomeric barrier preparation comprising a highly viscous, room temperature curing silicone composition, which forms a skin-friendly elastomer on curing, suitable for ostomy and wound dressing applications.

WO 2005/021058 A2 describes an adhesive composition comprising a hydrophilic silicone, a hydrophobic silicone, and optionally water absorbing material.

U.S. Patent Publication No. 2005/0266063 A1 describes an adhesive patch for transdermal drug delivery wherein the adhesive layer contains a mixture of hydrocarbon rubber and silicon-containing polymer.

U.S. Patent Publication No. 2005/0282977 A1 describes a pressure sensitive adhesive composition comprising a compatible blend of a silicone copolymer that reacts to form a gel and a non-reactive silicone pressure sensitive adhesive.

The above prior art disclosures are limited to silicone adhesive compostions and do not teach the how these adhesives can be used in ostomy and wound care applications.

### SUMMARY DESCRIPTION OF THE INVENTION

The object of this invention is an ostomy or wound care device for attachment to the body by a hot-melt pressure sensitive silicone adhesive.

Silicone pressure sensitive adhesives typically are comprised of two major components, a siloxane polymer and a silicate resin. The siloxane polymers have alternating silicone and oxygen atoms along their main chain. They cover a wide range of molecular weights, ranging from about 170 g/mol to more than 10⁶ g/mol. Examples of polymers used in silicone adhesives include polydimethylsiloxane, polymethylphenylsiloxane, polydimethyldiphenylsiloxane, and other silicone polymers including various organosiloxanes that are described generally as polysiloxanes. Silicones are generally hydrophobic, but they can be made less hydrophobic or more hydrophilic by modifying or copolymerizing them, for example, with alkylene oxides. Silicones and silicone adhesives are referred to herein interchangeably as polysiloxanes.

An example of a silicate resin is tetrakis (trimethylsiloxy) silicate. Either the polysiloxane or the resin or both may have silanol functionality. During adhesive manufacture, processing conditions are controlled so that a chemical reaction, for example, a condensation reaction or an addition reaction, occurs to yield a network of polysiloxane chains cross linked with the resin. The cross linking of the polysiloxane results in rheological properties necessary to achieve satisfactory tack, peel and cohesive properties.

Silicone adhesives of the kind described above may be obtained commercially in solvent or solvent-free forms. The solvent or solvent-free forms can be one-part or two-part adhesive systems depending on the curing system, or a combination of the two. In addition, these adhesives are also available as non-curing systems in a solvent.

Silicone pressure sensitive adhesives are generally regarded as soft and tacky adhesives which readily wet surfaces. As a consequence, the adhesion strength to a given surface increases with time leading to higher peel forces during removal of the adhesive than when applied initially. In the case of a skin adhesive, this is not desirable since the resulting high peeling force can remove and damage the skin. In order to maintain a level of adhesion to skin that is acceptable for both bonding and removing, it is necessary to balance the peel strength, cohesive strength, and tack of the adhesives. Since these properties are interrelated, it is not easy to achieve an optimal skin adhesive suitable for ostomy and wound care applications. Key parameters that control the properties of silicone adhesives include the molecular weight of the polymer, ratio of polymer to resin, and the degree of cross linking.

### DETAILED DESCRIPTION OF THE INVENTION

According to the present invention, an ostomy or wound care appliance device comprising an ostomy or wound care device for attachment to the body with an adhesive, said adhesive including a hot-melt silicone pressure sensitive adhesive composition and a reinforcing member, wherein said adhesive has the following characteristics:
i. limited cold flow;
ii. tack less than 2000 grams (ASTM D2729; contact force 100g; contact time 2 seconds; removal speed 0.1 mm/sec);
iii. storage (G') and loss (G") modulus between 1.0 x 10⁴ Pa and 5.0 x 10⁶ Pa at 5% strain and a frequency of 1 rad/s at 22 °C; and
iv. peel strength not less than 2.0 N/in or does not peel cleanly from the substrate (ASTM D3330; stainless steel substrate),

A commercially available hot-melt pressure sensitive silicone adhesive composition suitable according to the present invention is Bio-PSA® 7-4560 available from Dow Corning Corporation. Due to environmental concerns related to residues from flashing of organic solvents, the solvent free hot-melt silicone is preferred.

The reinforcing member may include fibers, meshes, screens, films, particulate fillers, thermoplastic polymers, wicking structures, porous structures, perforated structures, substrates, and combinations thereof.

Fibers may include natural or synthetic fibers such as cellulose, alginates, chitosan and its derivatives, rayon, polyesters, polyacrylonitriles, polyolefins, polyamides, polyurethanes, glass, and combinations thereof.

Meshes and screens may include natural or synthetic materials.

Films may include materials from natural or synthetic origin such as cellulose, alginates, chitosan and its derivatives, polyesters, polyacrylonitriles, polyolefins, diene elastomers, polyamides, polyurethanes, polyethers, polyvinyl alcohol, polyether block amides, polyimides, silicones, polyacrylates, polymethacrylates, ionomers, polyvinylacetate and its copolymers, polyvinylchloride, polyvinylidene chloride, fluorinated polymers, and combinations thereof.

Particulate fillers may include inorganic fillers such as talc, kaolin, silicas, silicates, borates, phosphates, nanoclays, calcium carbonate, alumina, laponite, zinc oxide, titanium dioxide, and combinations thereof.

Particulate fillers may include organic fillers such as hydrocolloids, proteins, latexes, waxes, superabsorbents, crosslinked polymers, and combinations thereof.

Thermoplastic polymers may include poly(vinyl-2-pyrrolidone) and its copolymers, polyvinyl ethers, polyureas, polyurethanes, silicone copolymers, polyethers, polyether block amides, polyamides, polyimides, polyesters, polyacrylonitriles, polyolefins, polyvinyl alcohol, vinlyaromatic block copolymers, polyacrylates, polymethacrylates, ionomers, polyvinyl chloride, polyvinylidene chloride, polyacrylic acid and its copolymers, polyvinyl acetate and its copolymers, ionomers, and combinations thereof.

The hot-melt silicone pressure sensitive adhesive composition may further include additives such as tackifiers, plasticizers, waxes, and combinations thereof.

Wicking structures may include nanotubes, fibers, surfactant-treated surfaces, and combinations thereof.

Perforated structures may include those created by printing processes such as stencil, screen, gravure, or offset, by coating processes such as spray or zone, by mechanical processes such as die-cutting or punching, and combinations thereof.

Porous structures may include those created by incorporating hollow polymer spheres, spheres filled with gas or liquid, processes known to produce open and close-celled foam, and combinations thereof.

Substrates may include foams, woven fabrics, nonwoven fabrics, elastomers, and combinations thereof.

Foams may include polyolefins, copolymers of vinyl acetate, silicones, polyurethanes, and combinations thereof.

Woven or non-woven fabrics may include any of the components of the fibers cited above.

Elastomers may include silicones, polyphosphazenes, polyolefins, diene elastomers, polyurethanes, vinylaromatic block copolymers, polyether block amides, polyacrylates, polymethacrylates, and combinations thereof.

Those skilled in the art will recognize that there are many mechanisms by which reinforcing members may be created by various means, and the description herein is not intended to limit the scope of the invention.

The hot-melt silicone pressure sensitive adhesive composition may be coated on the reinforcing member as a continuous or discontinuous coating.

The hot-melt silicone pressure sensitive adhesive composition may be coated on said reinforcing member as a pattern coating.

The hot-melt silicone pressure sensitive adhesive composition may further include a component for wet-surface adhesion such as a mucoadhesive component. The mucoadhesive component may include thiols, dihydroxyphenylalanine, polypeptides, amino acids, or combinations thereof.

### Examples

The silicone hot-melt pressure sensitive adhesive, Bio-PSA® 7-4560, was mixed with various additives in a twin blade mixer (Haake) at 205°F. The silicone was melted initially with constant mixing for about 10 minutes followed by the additive. The mixing was continued for an additional 10 minutes, after which the adhesive was scooped on to a fluorinated release liner. Examples with Kraton D1107 and Geniomer^{®} rubbers were mixed at 300°F. The rubber was first melted completely with constant mixing, followed by the addition of tackifier and oil in the case of D1107. Then the silicone was added.

Samples for tack and rheology measurements were made by pressing about 30 grams of the adhesive between fluorinated release liners at 180°F for 5 seconds at a pressure of 5000 psi.

Tack measurements were made using TAXT2i Texture Analyzer from TA Instruments. Measurements were made with a contact force of 100 grams, contact time of 2 seconds, and removal speed of 0.1 mm/sec.

Rheology measurements were made using an RDA III instrument from TA Instruments. A 25 mm diameter sample disc was cut and placed between two parallel plates of the rheometer. The test was carried out at a constant strain of 5% in the frequency range 100 rad/s to 0.1 rad/s at room temperature. The storage modulus G' and the loss modulus G" at a frequency of 1.0 rad/s is reported in Tables 1-5.

**Table 1**

| **TRADE NAME** | **DESCRIPTION** |
|---|---|
| Bio-PSA^{®} 7-4560 | Hot-melt silicone pressure sensitive adhesive from Dow Corning Corporation |
| CAB-O-SIL^{®} M-5 | Fumed silica from Cabot Corporation |
| SIPERNAT 2200 | Precipitated silica from Degussa Corporation |
| CLOISITE NA+ | Nanoclay from Southern Clay Products |
| Geniomer^{®} 60, 80, 200 | Polydimethylsiloxane/urea copolymer from Wacker Silicones |
| Kraton D1107 | Styrene-isoprene-styrene copolymer from Kraton Inc. |
| Arkon P-100 | Hydrocarbon resin from Arakawa Chemical Industries Ltd. |
| Kaydol oil | USP mineral oil from CP Witco Corporation |
| lrganox 1010 | Hindered phenolic antioxidant from Ciba-Geigy |
| Tech-O^{®} #11-080 | Colloidal oatmeal from Beacon Corporation |
| Pure Thix 1442 | Polyether-1 from Sud Chemie Inc. |

**Table 2**

| **Ingredients** | **A** | **B** | **C** | **D** | **E** |
|---|---|---|---|---|---|
| Bio-PSA^{®} 7-4560 | 100.0 | 90.9 | 83.2 | 77.0 | 90.0 |
| CAB-O-SIL^{®} M-5 | - | 9.1 | 8.3 | 23.0 | - |
| SIPERNAY 2200 | - | - | 8.5 | - | 5 |
| CLOISITE NA+ | - | - | - | - | 5 |
| Tack (g) | *361 +/- 11 | 561 +/- 54 | 567 +/- 20 | 323 +/- 32 | 364 +/- 16 |
| G' (10^4 Pa) @1rad/s,23°C and 5% strain | 1.0 | 3.4 | 7.2 | 32.0 | 1.7 |
| G"(10^4 Pa)@1rad/s,23° C and 5% strain | 1.2 | 4.2 | 8.6 | 32.0 | 2.1 |
| SS Peel strength (180-deg) N/in | 8.3 +/- 0.3 | Not measured | Not measured | Not measured | Not measured |

| | | | | | |
|---|---|---|---|---|---|
| * Adhesive did not release from probe | | | | | |

**Table 3**

| **Ingredients** | **A** | **B** | **C** | **D** | **E** | **F** |
|---|---|---|---|---|---|---|
| Bio-PSA^{®} 7-4560 | 90.0 | 70.0 | 53.0 | 70.0 | 80.0 | 70.0 |
| Geniomer^{®} 60 | 10.0 | 30.0 | 46.0 | - | - | - |
| Geniomer^{®} 80 | - | - | - | 30.0 | 20.0 | - |
| Geniomer^{®} 200 | - | - | - | - | - | 30.0 |
| Tack (g) | 375 +/- 57 | 678 +/- 63 | 251 +/- 5 | 345 +/- 112 | 253 +/- 16 | 417 +/- 22 |
| G' (10^4 Pa) @ 1.0 rad/s, 23°C and 5% strain | 3.5 | 3.3 | 13.0 | 2.3 | | 2.9 |
| G" (10^4 Pa) @ 1.0 rad/s, 23°C and 5% strain | 2.6 | 2.4 | 4.3 | 1.5 | | 2.3 |
| G' (10^4 Pa) @ 0.1 rad/s, 23°C and 5% strain | 1.7 | 1.6 | 9.4 | 1.0 | | 1.2 |
| SS Peel strength (180-deg) N/in | Not measured | 3.3 +/- 0.5 | Not measured | Not measured | 4.6 +/- 0.5 | 3.5 |

**Table 4**

| **Ingredients** | **A** |
|---|---|
| Bio-PSA^{®} 7-4560 | 23.6 |
| Kraton D1107 | 17.7 |
| Arkon P-100 | 53.0 |
| Kaydol oil | 5.7 |
| Irganox 1010 | 0.5 |
| Tack (g) | 746 +/- 88 |
| G' (10^4 Pa) @1rad/s,23°C and 5% strain | 5.5 |
| G" (10^4 Pa)@1rad/s,23°C and 5% strain | 16.0 |

**Table 5**

| **Ingredients** | **A** | **B** |
|---|---|---|
| Bio-PSA^{®} 7-4560 | 67.9 | 90.0 |
| Tech-O^{®} #11-080 | 33.0 | - |
| Pure Thix 1442 | - | 10.0 |
| Tack (g) | 435 +/- 29 | 140 +/- 12 |
| G' (10^4 Pa) @1rad/s,23°C and 5% strain | 1.6 | 21.0 |
| G" (10^4 Pa)@1rad/s,23°C and 5% strain | 1.8 | 14.0 |

In addition, about 30 grams of Bio-PSA® 7-4560 was pressed between two layers of nonwoven fabric, which were placed between two fluorinated release liners, at 180°F for 2 seconds at a pressure of 1 bar. The adhesive flowed through the nonwoven fabrics and the resulting surface had a tack of 504 +/- 58 grams, storage modulus G' value of 1.3 x 10⁴ Pa, and loss modulus G" value of 1.4 x 10⁴ Pa at 1.0 rad/sec and 23°C.

## Claims

1. An ostomy or wound care appliance comprising an ostomy or wound care device for attachment to the body with an adhesive, said adhesive including a hot-melt silicone pressure sensitive adhesive composition and a reinforcing member, wherein said adhesive has the following characteristics:
i. limited cold flow;
ii. tack less than 2000 grams (ASTM D2729; contact force 100g; contact time 2 seconds; removal speed 0.1 mm/sec);
iii. storage (G') and loss (G") modulus between 1.0 x 10⁴ Pa and 5.0 x 10⁶ Pa at 5% strain and a frequency of 1 rad/s at 22°C
iv. peel strength not less than 2.0 N/in or does not peel cleanly from the substrate (ASTM D3330; stainless steel substrate)

2. The ostomy or wound care appliance of claim 1, wherein the said reinforcing member is selected from the group consisting of fibers, meshes, screens, films, particulate fillers, thermoplastic polymers, wicking structures, perforated structures, porous structures, substrates, and combinations thereof.

3. The ostomy or wound care appliance of claim 2, wherein said fibers include natural or synthetic fibers such as cellulose, alginates, chitosan and its derivatives, rayon, polyesters, polyacrylonitriles, polyolefins, polyamides, polyurethanes, glass, and combinations thereof.

4. The ostomy or wound care appliance of claim 2 or 3, wherein said meshes and screens include natural or synthetic materials, and combinations thereof.

5. The ostomy or wound care appliance of claim 2, 3 or 4, wherein the said films include materials from natural or synthetic origin such as cellulose, alginates, chitosan and its derivatives, polyesters, polyacrylonitriles, polyolefins, diene elastomers, polyamides, polyurethanes, polyethers, polyvinyl alcohol, polyether block amides, polyimides, silicones, polyacrylates, polymethacrylates, ionomers, polyvinylacetate and its copolymers, polyvinylchloride, polyvinylidene chloride, fluorinated polymers, and combinations thereof.

6. The ostomy or wound care appliance of claim 2, 3, 4 or 5, wherein the said particulate fillers include inorganic fillers such as talc, kaolin, silicas, silicates, borates, phosphates, nanoclays, calcium carbonate, alumina, laponite, zinc oxide, titanium dioxide, and combinations thereof.

7. The ostomy or wound care appliance of any of claims 2 to 6, wherein said particulate fillers include organic fillers such as hydrocolloids, proteins, latexes, waxes, superabsorbents, crosslinked polymers, and combinations thereof.

8. The ostomy or wound care appliance of any of claims 2 to 7, wherein the thermoplastic polymers include poly(vinyl-2-pyrrolidone) and its copolymers, polyvinyl ethers, polyureas, polyurethanes, silicone copolymers, polyethers, polyether block amides, polyamides, polyimides, polyesters, polyacrylonitriles, polyolefins, polyvinyl alcohol, vinlyaromatic block copolymers, polyacrylates, polymethacrylates, ionomers, polyvinyl chloride, polyvinylidene chloride, polyacrylic acid and its copolymers, polyvinyl acetate and its copolymers, ionomers, and combinations thereof.

9. The ostomy or wound care appliance of any preceding claim, wherein the said hot-melt silicone pressure sensitive adhesive composition further includes additives such as tackifiers, plasticizers, waxes, and combinations thereof.

10. The ostomy or wound care appliance of claim 2 or any claim dependent thereon, wherein the said wicking structures include nanotubes, fibers, surfactant-treated surfaces, and combinations thereof.

11. The ostomy or wound care appliance of claim 2 or any claim dependent thereon, wherein the said perforated structures include those created by printing processes such as stencil, screen, gravure, or offset, by coating processes such as spray or zone, by mechanical processes such as die-cutting or punching, and combinations thereof.

12. The ostomy or wound care appliance of claim 2 or any claim dependent thereon, wherein said porous structures include those created by incorporating hollow polymer spheres, spheres filled with gas or liquid, processes known to produce open and close-celled foam, and combinations thereof.

13. The ostomy or wound care appliance of claim 2 or any claim dependent thereon, wherein said substrates include foams, woven fabrics, nonwoven fabrics, elastomers, and combinations thereof.

14. The ostomy or wound care appliance of claim 13, wherein the said foams include polyolefins, copolymers of vinyl acetate, silicones, polyurethanes, and combinations thereof.

15. The ostomy or wound care appliance of claim 13 or 14, wherein the said woven or non-woven fabrics include any of the components of the fibers cited above.

16. The ostomy or wound care appliance of claim 13, 14 or 15, wherein the said elastomers include silicones, polyphosphazenes, polyolefins, diene elastomers, polyurethanes, vinylaromatic block copolymers, polyether block amides, polyacrylates, polymethacrylates, and combinations thereof.

17. The ostomy or wound care appliance of any preceding claim, wherein the said hot-melt silicone pressure sensitive adhesive composition further includes additives such as tackifiers, plasticizers, waxes, and combinations thereof

18. The ostomy or wound care appliance of any preceding claim, wherein the said hot-melt silicone pressure sensitive adhesive composition is coated on the reinforcing member as a continuous or discontinuous coating, and combinations thereof.

19. The ostomy or wound care appliance of any preceding claim, wherein the said hot-melt silicone pressure sensitive adhesive composition is coated on said reinforcing member as a pattern coating.

20. The ostomy or wound care appliance of any preceding claim, wherein the said hot-melt silicone pressure sensitive adhesive composition further includes a component for wet-surface adhesion such as a mucoadhesive component.

21. The ostomy or wound care appliance of claim 20, wherein the mucoadhesive component includes thiols, dihydroxyphenylalanine, polypeptides, amino acids, or combinations thereof.
